# EUROPEAN PATENT SPECIFICATION

(11) **EP 1 429 731 B1**
(45) Date of publication and mention of the grant of the patent: **03.01.2007**
(21) Application number: 02775863.0
(22) Date of filing: 19.09.2002
(51) Int. Cl.: A61K 9/14, A61K 38/28

(54) **NANOPARTICULATE INSULIN FORMULATIONS**
NANOPARTIKELZUSAMMENSETZUNGEN ENTHALTEND INSULIN
PREPARATIONS D'INSULINE NANOPARTICULAIRES

(30) Priority: 19.09.2001 US 323459 P
(43) Date of publication of application: 23.06.2004
(73) Proprietor: Elan Pharma International Limited, Shannon, County Clare (IE)
(72) Inventor: McGURK, Simon, L., King of Prussia, PA 19406 (US); MERISKO-LIVERSIDGE, Elaine, West Chester, PA 19380 (US); O'MAHONEY, Daniel, Blackrock, Co. Dublin (IE); WEIDERHOLD, Amy, Hatfield, PA 19440 (US); RAOOF, Araz, 1950 Kraainem (BE)
(74) Representative: Pohlman, Sandra M.
(86) International application number: PCT/US2002/029679
(87) International publication number: WO 2003/024425

(56) References cited:
- EP-A- 0 499 299
- WO-A-00/27363
- WO-A-00/51572
- WO-A-01/26635
- WO-A-99/02665
- US-A- 5 470 583
- US-A1- 2002 110 597
- US-B1- 6 267 989
- US-B1- 6 316 029
- US-B1- 6 375 986

## Description

### Field of the Invention

The present invention is directed to nanoparticulate compositions comprising insulin, having adsorbed to the surface of the insulin particles at least one surface stabilizer.

### Background of the Invention

### A. Background Regarding Nanoparticulate Compositions

Nanoparticulate compositions, first described in U.S. Patent No. 5,145,684 ("the '684 patent"), are particles consisting of a poorly soluble active agent having adsorbed onto the surface thereof a non-crosslinked surface stabilizer. The '684 patent also describes methods of making such nanoparticulate compositions. Nanoparticulate compositions are desirable because with a decrease in particle size, and a consequent increase in surface area, a composition is rapidly dissolved and absorbed following administration. The '684 patent does not teach or suggest nanoparticulate compositions comprising peptides or insulin.

Methods of making nanoparticulate compositions are described, for example, in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,653 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging:" 5,518,738 for "Nanoparticulate NSAID Formulations;" 5,521,218 for "Nanoparticulate lododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 and WO 99/02665 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" and 6,432,381 for "Methods for Targeting Drug Delivery to the Upper and/or Lower Gastrointestinal Tract," all of which are specifically incorporated by reference. In addition, U.S. Patent Application No. 20020012675 A1, published on January 31, 2002, for "Controlled Release Nanoparticulate Compositions," describes nanoparticulate compositions.

Nanoparticulate peptides and proteins are referenced in U.S. Patent No. 6,375,986 for "Solid Dose Nanoparticulate Compositions Comprising a Synergistic Combination of a Polymeric Surface Stabilizer and Dioctyl Sodium Sulfosuccinate," U.S. Patent No. 6,428,814 and WO 01/26635 for "Bioadhesive Nanoparticulate Compositions Having Cationic Surface Stabilizers," U.S. Patent Application No. 20020012675 A1 for "Controlled Release Nanoparticulate Compositions," WO 00/51572 for "Use of PEG-Derivatized Lipids as Surface Stabilizers for Nanoparticle. Compositions," published on September 8, 2000, and WO 00/53164 for "Methods for Preventing Crystal Growth and Particle Aggregation in Nanoparticulate Compositions, published on September 14, 2000. Furthermore, WO 00/27363 discloses nanoparticulate aerosol formulations and methods of making such formulations and EP 0 499 299 relates to surface modified drug nanoparticles. However, none of these references teach or suggest that the peptide or protein can be insulin.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Methods;" and 5,776,496, for "Ultrasmall Porous Particles for Enhancing Ultrasound Back Scatter." None of these references relates to a nanoparticulate insulin composition.

### B. Background Regarding Delivery of Peptides and Proteins

Delivery of peptides and proteins has long been a problem because of high toxicity, poor bioavailability, and degrading of the peptide or protein when formulated for administration or application. Numerous approaches to formulating peptide and protein compositions have been developed. For example, U.S. Patent No. 5,889,110 discloses microparticles comprising a salt formed from a cation derived from a peptide containing one or more basic groups and an anion derived from a carboxy-terminated polyester. The composition is prepared from a stoichiometric equivalent of the polyester carboxylic acid end groups relative to the basic peptide groups, obtainable by a complicated and expensive process.

Such a process comprises: (i) dissolving the basic peptide and carboxy-terminated polyester in a first solvent in which both the peptide and the polyester are soluble to form a first solution; (ii) freezing the first solution at high speed to form a frozen mixture; (iii) freeze-drying the frozen mixture to remove the first solvent to form a freeze-dried product; (iv) dispersing the freeze-dried product into a second solvent which is a solvent for the polyester and a non-solvent for the peptide to form a second solution containing the peptide/polyester salt; and (v) removing the second solvent from the second solution by spray-drying, spray-congealing, evaporation, or phase separation coacervation to form a microparticulate solid product.

U.S. Patent No. 5,354,562 discloses a process for preparing micronized polypeptide drugs in a powder form suitable for aerosol administration or for use in injectable suspensions by lyophilization followed by jet milling. U.S. Patent No. 6,051,694 discloses a method of size reduction of proteins comprising contacting the solid protein with a critical fluid in which the protein is essentially insoluble, followed by depressurization of the protein and critical fluid mixture.

None of the prior art methods provide nanoparticulate insulin compositions. In fact, attempts to mill peptides such as insulin have resulted in a change of conformation of the peptides. For example, Leung et al., *J. Pharm. Sci.,87*:501-7 (1998), disclose transformation of the crystal structure of aspartame hemihydrate into a different polymorph upon ball milling of the peptide. This is not surprising, as peptides and proteins can be labile to milling conditions, e.g., shear, pressure, and temperature.

There remains a need in the art for nanoparticulate insulin formulations that would facilitate insulin delivery in high dosages with consistent and effective activity and other advantages in comparison to any conventional preparations presently available. In accordance with the present invention, such a preparation of insulin is provided.

### Summary of the Invention

The present invention is directed to the surprising and unexpected discovery that stable nanoparticulate compositions of insulin can be made. The nanoparticulate compositions, which comprise insulin and at least one surface stabilizer adsorbed to the surface thereof, are advantageous in comparison to prior art insulin preparations in that they possess both a rapid onset of activity and prolonged activity.

The invention is further directed to methods of making nanoparticle insulin compositions and pharmaceutical dosage forms containing them. The method of preparing the insulin compositions comprises contacting insulin, *e.g.* insoluble bovine insulin or recombinant insulin, with at least one surface stabilizer for a time and under conditions sufficient to provide a stable nanoparticulate composition. The particle size of insulin is reduced by milling or homogenization. The one or more surface stabilizers can be contacted with the insulin either before, during, or after size reduction thereof.

The present invention is further related to preparations to be used in the treatment of conditions requiring insulin therapy.

### Brief Description of the Figures

- FIG 1:: Are scanning electron microscope images at 5,000 magnification of insulin particles before and after milling in accordance with the method of the present invention;
- FIG 2:: Shows the results of a reducing SDS PAGE gel of the supernatant and pellet of a nanoparticulate insulin sample in two concentrations to determine whether milling caused a loss of insulin;
- FIG 3:: Shows the results of a reducing SDS PAGE gel of the pellet of a nanoparticulate insulin sample in two concentrations to determine whether milling caused a loss of insulin;
- FIG 4:: Shows the pharmacokinetic parameters of insulin resulting from injection of nanoparticulate insulin samples via intramuscular, subcutaneous and intraperitoneal routes in comparison to a control insulin solution:
- FIG 5:: Is a bar graph illustrating the AUC (µU/ml.h) of insulin from injection of nanoparticulate insulin samples via subcutaneous, intramuscular and intraperitoneal routes in comparison to a control insulin solution;
- FIG. 6:: Is a plot showing blood insulin concentration over time following injection of nanoparticulate insulin samples via subcutaneous, intramuscular and intraperitoneal routes in comparison to a reference insulin solution;
- FIG. 7:: Is a plot showing blood glucose concentration over time following injection of nanoparticulate insulin samples via subcutaneous, intramuscular and intraperitoneal routes in comparison to a reference insulin solution; and
- FIG. 8: Is a plot showing the change in blood glucose concentration over time following injection of nanoparticulate insulin samples via subcutaneous, intramuscular and intraperitoneal routes in comparison to a reference insulin solution.

### Detailed Description of the Invention

The present invention is directed to compositions of nanoparticulate insulin and methods for their preparation and use in insulin therapy. Prior to the present invention, it was known that crystalline drugs could be formulated into nanoparticulate compositions by the method taught in the '684 patent. However, as discussed above, formulations of nanoparticulate insulin were not contemplated, partly due to the difficulty in retaining the insulin conformational structure upon milling to a particle size in the nanometer region. This is significant, as a change in conformation of insulin can correlate with a loss of activity.

Nanoparticulate insulin, prepared in accordance to the present invention, comprises at least one surface stabilizer that is adsorbed to the insulin particles. The surface stabilizer acts as a steric barrier to other insulin particles thereby preventing agglomeration and particle size growth. This results in a stable nanoparticulate composition, in which the particle size of the composition does not significantly increase over time via solubilization and recrystallization or agglomeration. Individually adsorbed molecules of the surface stabilizer are essentially free of intermolecular crosslinkages.

Insulin is a 5.8 kD protein hormone important in regulation of fuel metabolism. Secretion of insulin by the β cells of the pancreas is stimulated by glucose and the parasympathetic nervous system. Insulin promotes the dephosphorylation of key interconvertible enzymes. One consequence is to stimulate glycogen synthesis in both muscle and liver and to suppress gluconeogenesis by the liver. Insulin also accelerates glycolysis in the liver, which in turn increases the synthesis of fatty acids. The entry of glucose into muscle and adipose cells is promoted by insulin. The abundance of fatty acids and glucose in adipose tissue results in the synthesis and storage of triacylglycerols. The action of insulin also extends to amino acid and protein metabolism. Insulin promotes the uptake of branched-chain amino acids by muscle, which favors a building up of muscle protein. In sum, insulin has a general stimulating effect on protein synthesis.

Three different compositions of insulin are available: soluble, insoluble, and a mixture thereof. The difference among these compositions is in the observed bioavailability at physiological conditions. Insoluble insulin, and a mixture of soluble and insoluble insulin, have a slow onset of activity, about 2 hours, but a prolonged duration that extends for up to about 24 and 13-14 hours, respectively. Soluble insulin has a more rapid onset of activity, about 30 minutes, but with only about six hours duration of activity. The nanoparticle insulin of the present invention is superior to prior art insulin compositions in that it displays a rapid onset of activity, such as that found with soluble insulin, and it exhibits a prolonged duration of activity, such as that found in insoluble and soluble-insoluble insulin mixtures.

The insulin composition can be formulated for administration via, for example, oral, pulmonary, nasal, parenteral, rectal, local, buccal, or topical administration.

The insulin particles can be in the form of crystalline particles, semi-crystalline particles, semi-amorphous particles, amorphous particles, or a mixture thereof.

The concentration of insulin in the compositions of the invention can vary from about 99.5% to about 0.001%, from about 95% to about 0.1%, or from about 90% to about 0.5%, by weight, based on the total combined dry weight of the insulin and at least one surface stabilizer, not including other excipients.

### A. Useful Surface Stabilizers

Suitable surface stabilizers utilized in preparing the subject nanoparticle insulin are preferably selected from known organic and inorganic pharmaceutical excipients. Two or more surface stabilizers may be used in combination. The surface stabilizers protect insulin from degradation and potential protease cleavage in addition to preventing agglomeration and particle size growth as described above.

Such organic and inorganic pharmaceutical excipients include various polymers, low molecular weight oligomers, natural products, and surfactants. Preferred surface stabilizers include nonionic and ionic surfactants (e.g., cationic and anionic surfactants). Representative examples of surface stabilizers include cetyl pyridinium chloride, gelatin, lecithin (phosphatides), dextran, glycerol, cholesterol, tragacanth, stearic acid, its salts and its esters, cetomacrogol emulsifying wax, sorbitan esters, polyoxyethylene alkyl ethers, polyoxyethylene castor oil derivatives, polyoxyethylene sorbitan fatty acid esters, e.g., the commercially available Tweens® such as Tween 80® (ICI Specialty Chemicals); polyethylene glycols, e.g., Carbowaxs 3350® and 1450®, and Carbopol 934® (Union Carbide), dodecyl trimethyl ammonium bromide, polyoxyethylene stearates, colloidal silicon dioxide, sodium dodecylsulfate, carboxymethylcellulose calcium, hydroxypropyl celluloses (e.g., HPC, HPC-SL, and HPC-L), hydroxypropyl methylcellulose (HPMC), carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol (PVA), polyvinylpyrrolidone (PVP), 4-(1,1,3,3-tetramethylbutyl)-phenol polymer with ethylene oxide and formaldehyde (also known as tyloxapol), poloxamers, e.g., Pluronics F68® and F108®, which are block copolymers of ethylene oxide and propylene oxide; poloxamines, e.g., Tetronic 908®, also known as Poloxamine 908®, which is a tetrafunctional block copolymer derived from sequential addition of propylene oxide and ethylene oxide to ethylenediamine (BASF Corporation, Parsippany, N.J.)); a charged phospholipid such as dimyristoyl phophatidyl glycerol, dioctylsulfosuccinate (DOSS); Tetronic 1508® (T-1508) (BASF Corporation), dialkylesters of sodium sulfosuccinic acid (e.g., Aerosol OT®, which is a dioctyl ester of sodium sulfosuccinic acid (American Cyanamid)); sodium lauryl sulfate (SLS), such as Duponol P® (DuPont); Tritons X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-lOG® or Surfactant 10-G® (Olin Chemicals, Stamford, CT); Crodestas SL-40® (Croda, Inc.); poly (2-methacryloxyethyltrimethylammonium bromide) (S1001); poly(N-vinylpyrrolidone/2-dimethylaminoethyl methacrylate) di methylsulphate quaternary (S1002); poly(2-methylacryloxyamidopropyltrimethylammonium chloride)(S1004); sodium deoxycholic acid; vitamin E derivatized with polyethylene glycol (PEG), a phospholipid derivatized with PEG, cholesterol derivatized with PEG, a cholesterol derivative derivatized with PEG, vitamin A derivatized with PEG, benzalkonium chloride (BKC), centrimide (a quaternary ammonium compound), Span® 20 (sorbitan monolaurate), Plasdone® S630, (a random copolymer of polyvinylpyrrolidone (PVP) and vinyl acetate in a 60:40 ratio), hexyldecyltrimethylammonium bromide (HDMAB), triblock copolymers of the structure -(-PEO)-(-PBO-)--(-PEO-)- (known as B20-5000), poly (2-methacryloxyethyltrimethylammonium bromide) (S1001), poly(N-vinylpyrrolidone/2-dimethylaminoethyl methacrylate) di methylsulphate quaternary (S1002), poly(2-methylacryloxyamidopropyltrimethylammonium chloride) (S1004), random copolymers of PVP and vinyl acetate, lysozyme, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅ dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336^{™}), POLYQUAT 10^{™}, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quaternized polyoxyethylalkylamines, MIRAPOL^{™} and ALKAQUAT^{™} (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, *Cationic Surfactants: Analytical and Biological Evaluation* (Marcel Dekker, 1994); P. and D. Rubingh (Editor), *Cationic Surfactants: Physical Chemistry* (Marcel Dekker, 1991); and J. Richmond, *Cationic Surfactants: Organic Chemistry,* (Marcel Dekker, 1990).

Particularly preferred nonpolymeric primary stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quaternium 22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000). The surface stabilizers are commercially available and/or can be prepared by techniques known in the art starting from known materials.

The concentration of the at least one surface stabilizer can vary from about 0.001 to about 99.5%, from about 0.1% to about 95%, and from about 0.5% to about 90%, by weight, based on the total combined dry weight of the insulin and at least one surface stabilizer, not including other excipients.

### B. Particle Size of the Insulin Particles

Preferably, the insulin nanoparticles of the invention have an effective average particle size of less than about 5 microns, less than about 4 microns, less than about 3 microns, less than about 2 microns, less than about 1500 nm, less than about 1 micron, less than about 800 nm, less than about 700 nm, less than about 600 nm, less than about 500 nm, less than about 400 nm, less than about 300 nm, less than about 200 nm, less than about 100 nm, less than about 75 nm, or less than about 50 nm, as measured by light-scattering methods or other methods accepted in the art. By "an effective average particle size of less than about 5 microns" it is meant that at least about 50% of the insulin particles have a weight average particle size of less than about 5 microns when measured by light scattering or other conventional techniques. In addition, in other embodiments of the invention, at least about 70%, about 90%, or about 95% of the insulin particles have an effective average particle size of less than about 5 microns, less than about 4 microns, less than about 3 microns, less than about 2 microns, less than about 1500 nm, less than about 1 micron, less than about 800 nm, less than about 700 nm, less than about 600 nm, less than about 500 nm, less than about 400 nm, less than about 300 nm, less than about 200 nm, less than about 100 nm, less than about 75 nm, or less than about 50 nm.

### C. Methods of Making Nanoparticulate Insulin Compositions

Nanoparticulate active agent compositions can be made using, for example, milling, or homogenization techniques. Exemplary methods of making nanoparticulate compositions are described in U.S. Patent No. 5,145,684. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation".

The optimal effective average particle size of the invention can be obtained by controlling the process of particle size reduction, such as by controlling the milling time and the amount of surface stabilizer added. Particle size growth and particle aggregation can also be minimized by milling and/or storing the composition under colder temperatures.

The nanoparticulate insulin dispersions made via, for example, milling or homogenization can be utilized in solid, semi-solid, or liquid dosage formulations, such as controlled release formulations, solid dose fast melt formulations, aerosol formulations, lyophilized formulations, tablets, solid lozenge, capsules, powders, liquids for injection, etc.

### 1. Milling to Obtain Nanoparticulate Insulin Compositions

Milling insulin to obtain a nanoparticulate composition comprises dispersing insulin particles in a liquid dispersion medium in which insulin is poorly soluble, followed by applying mechanical means in the presence of grinding media to reduce the particle size of the insulin to the desired effective average particle size. The dispersion medium can be, for example, water, safflower oil, ethanol, t-butanol, glycerin, polyethylene glycol (PEG), hexane, or glycol.

The insulin particles can be reduced in size in the presence of at least one surface stabilizer. Alternatively, the insulin particles can be contacted with one or more surface stabilizers after attrition. It is preferred, however, to disperse the insulin in the liquid dispersion medium in the presence of the at least one surface stabilizer as an aid in wetting of the insulin particles. Other compounds, such as a diluent, can be added to the insulin/surface stabilizer composition during the size reduction process. Dispersions can be manufactured continuously or in a batch mode.

### 2. Homogenization to Obtain Insulin Nanoparticulate Compositions

Exemplary homogenization methods of preparing active agent nanoparticulate compositions are described in U.S. Patent No. 5,510,118, for "Process of Preparing Therapeutic Compositions Containing Nanoparticles."

Such a method comprises dispersing insulin particles in a liquid dispersion medium, followed by subjecting the dispersion to homogenization to reduce the particle size of the insulin to the desired effective average particle size. The insulin particles can be reduced in size in the presence of at least one surface stabilizer. Alternatively, the insulin particles can be contacted with one or more surface stabilizers either before or after particle size reduction. It is preferred, however, to disperse the insulin in the liquid dispersion medium in the presence of the at least one surface stabilizer as an aid to wetting of the insulin particles. Other compounds, such as a diluent, can be added to the insulin/surface stabilizer composition either before, during, or after the size reduction process. Dispersions can be manufactured continuously or in a batch mode.

### D. Excipients

Pharmaceutical compositions in accordance with the present invention include nanoparticulate insulin compositions formulated together with one or more non-toxic, physiologically acceptable carriers, adjuvants, or vehicles, collectively referred to as carriers. Such carriers may comprise physiologically acceptable sterile aqueous or nonaqueous solutions, dispersions, suspensions or emulsions, and sterile powders for reconstitution into such preparations. Examples of suitable aqueous and nonaqueous carriers, diluents, solvents, or vehicles include water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions and by the use of surfactants.

The nanoparticulate compositions may also contain adjuvants such as preserving, wetting, emulsifying, and dispensing agents. Prevention of the growth of microorganisms can be ensured by various antibacterial and antifungal agents, such as potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride, parabens, chlorobutanol, phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

In one embodiment of the invention, the nanoparticulate compositions of the invention are intended for parenteral administration to humans and animals by known routes of parenteral administration, *i.e*., intravenous, intramuscular, subcutaneous, or intraperitoneal. Other dosage forms can also be utilized, such as aerosols, tablets, etc.

Actual dosage levels of the nanoparticulate insulin compositions of the invention are given as standardized units of insulin as is the case with conventional insulin preparations and the dosage administered will depend on recognized factors such as the time of administration and the body weight, general health, age, sex, and diet of the patient.

The following examples are given to illustrate the present invention. It should be understood, however, that the invention is not to be limited to the specific conditions or details described in these examples.

### Example 1

The purpose of this example was to prepare nanoparticulate insulin formulations using low energy milling techniques.

Insulin and one or more surface stabilizers, in the amounts shown in Table 1, were mixed with water to form a pre-milling slurry. This slurry was then added to a sealable vessel and rotated for from 1 to 4 days at a pre-set rotational speed (typically 100-200 rpm) on a roller mill using high wear zirconia grinding media (Tosoh Corporation, Tokyo, Japan) with a diameter of 0.8 mm. This low energy milling technique relies upon gravitational grinding mechanisms to break the particle size down, hence the use of heavy ceramic media.

Particle size distributions of the resultant insulin compositions were determined using a Horiba LA-910 light-scattering particle size analyzer (Horiba Instruments, Irvine, CA). The results shown in Table 1 were determined at 24 hours post milling. It is to be noted that the concentration of insulin in this Example was 5%, but could have been, for example, 1%, 2%, 3%, or 10% or higher.

The surface stabilizers used include: (1) poly (2-methacryloxyethyl-trimethylammonium bromide) (S1001); (2) poly(N-vinylpyrrolidone/2-dimethyl-aminoethyl methacrylate) dimethylsulphate quarternary (S 1002); (3) poly(2-methylacryloxyamidopropyltrimethylammonium chloride) (S1004); (4) Pluronic® F68; (5) hydroxypropyl cellulose (HPC); (6) sodium deoxycholic acid; (7) vitamin E derivatized with polyethylene glycol (PEG); and (8) Tween® 80.

| **TABLE 1** | | | | |
|---|---|---|---|---|
| **Low Energy Ball-Milled Nanoparticulate Insulin Formulations (Particle Size 24 hrs after Milling)** | | | | |
| **Exp No.** | **% Drug** | **% of Stabilizer** | **Mean Particle Size** @ **24 hours after milling (nm)** | **Particle size of 90%** @ **24 hours after milling (nm)** |
| 1 | 5.0% insulin | 0.0 | 4198 | 7092 |
| | | | (1344 after 1 min sonication) | (3104 after 1 min sonication) |
| 2 | 5.0% insulin | 1% F68 0.1% | 165 | 266 |
| | | Sodium Deoxycholic Acid | (144 after 1 min sonication) | (193 after 1 min sonication) |
| 3 | 5.0% insulin | 1% PVP k17 | 199 | 279 |
| | | 0.1% Sodium Deoxycholic Acid | (163 after 1 min sonication) | (219 after 1 min sonication) |
| 4 | 5.0% insulin | 1% PVP k29-32 | 243 | 364 |
| | | 0.1% Sodium Deoxycholic Acid | (171 after 1 min sonication) | (250 after 1 min sonication) |
| 5 | 5.0% insulin | 1% S1004 | 252 | 362 |
| 6 | 5.0% insulin | 1% S1001 | 282 | 401 |
| | | | (258 after 1 min sonication) | (380 after 1 min sonication) |

The measurements reported in Table 2 were taken at one week post milling to determine stability for a representative group of the nanoparticulate compositions shown in Table 1. In several instances, the particle size of the composition was smaller after one week than at 24 hours post milling. While not being bound to any one theory, it is believed that the stabilizer and drug particle may go through a thermodynamic relaxation period. Another theory is that in conjunction with relaxation or separately therefrom, there are interactions between the insulin particle and the stabilizer. Further, a phenomenon may be occurring at the surface of the insulin particle that is similar to the use of cationic stabilizers to complex and condense DNA in solution, i.e. the stabilizer interaction may cause a slight condensation on the surface of the insulin particle, resulting in the observed reduction in particle size.

| **TABLE 2** | | | | | | |
|---|---|---|---|---|---|---|
| **Low Energy Ball-Milled Nanoparticulate Insulin Formulations (Particle Size 24 hrs and 1 Week After Milling)** | | | | | | |
| **Exp No.** | **% Drug** | **% of Stabilizer** | **Mean Particle Size @ 24 hours after milling (nm)** | **Particle size of 90% @ 24 hours after milling (nm)** | **Mean Particle Size @ 1 week after milling (nm)** | **Particle size of 90 % @ 1 week after milling (nm)** |
| 4 | 5.0% insulin | 1% PVP k29-32 | 243 | 364 | 212 | 301 |
| | | 0.1% Sodium Deoxycholic Acid | (171 after 1 min sonication) | (250 after 1 min sonication) | (165 after 1 min sonication) | (222 after 1 min sonication) |
| 5 | 5.0% insulin | 1% S1004 | 252 | 362 | 263 | 364 |
| 6 | 5.0% insulin | 1% S1001 | 282 | 401 | 303 | 450 |
| | | | (258 after 1 min sonication) | (380 after 1 min sonication) | (266 after 1 min sonication) | (384 after 1 min sonication) |
| 7 | 5.0% insulin | 1% S1001 | 521 | 1004 | 498 | 905 |

### Example 2

The purpose of this example was to prepare nanoparticulate insulin formulations using high energy milling conditions.

Samples of insulin were prepared using a high energy attrition media mill, NanoMill™ (Elan Drug Delivery, King of Prussia, PA). A high energy mill is designed to apply a much higher rotational velocity to the particulate dispersion (100-6000 rpm, typically 5000 rpm), than that used in low energy milling processes. The elevated rotational velocity imparts very high shear conditions within the milling chamber. It is this shear force which causes the particle size reduction.

The media used in this milling technology is a much lighter, highly crosslinked polystyrene media. For preparation of nanoparticulate insulin compositions described in Table 3, 500 µm media was used. Other media sizes that could be used range from 50 µm to 500 µm. The samples were milled from about 30 minutes up to about 3 hours. Particle size distributions were determined using a Horiba LA-910 light-scattering particle size analyzer (Horiba Instruments, Irvine, CA). Particle size was determined for all three compositions following milling, for Sample 2 at 24 hours following milling, and for Samples 1 and 2 at 1 week after milling. The results are shown in Table 3.

| **TABLE 3** | | | | | | | | |
|---|---|---|---|---|---|---|---|---|
| **HIGH ENERGY MILLED NANOPARTICULATE INSULIN FORMULATIONS** | | | | | | | | |
| **Exp. No.** | **% of Insulin** | **% of Stabilizer** | **Mean Particle Size (nm)** @ **harvest** | **Particle Size (nm) of 90%** @ **harvest** | **Mean Particle Size (nm)** @ **24 hrs after milling** | **Particle Size of 90% (nm)** @ **24 hrs after milling** | **Mean Particle Size (nm)** @ **1 week after milling** | **Particle size of 90% (nm)** @ **1 week after milling** |
| 1 | 4.0% | 1% S1001 | 255 | 378 | | | 297 | 436 |
| | | | 234 | 353 | | | 247 | 372 |
| 2 | 2.5% | 1%S1004 | 282 | 381 | 284 | 382 | 232 | 329 |
| | | | 239 | 341 | 275 | 370 | 217 | 207 |

These results demonstrate the ability to produce nanoparticulate insulin compositions which maintain stability and perhaps even improve upon storage using high energy milling techniques. Most importantly, the results demonstrate that, upon storage, little or no aggregation is observed. Thus, the data show the ability to consistently prepare nanoparticulate insulin formulations which maintain their integrity upon storage.

### Example 3

The purpose of this example is to examine nanoparticle insulin prepared in accordance with the invention and test the stability of the compositions over an extended storage period.

Nanoparticulate insulin was prepared in accordance with the procedure of Example 2. Milling was carried out until the insulin particles were found to have a mean particle size of 100 nm, with 90% of the particles less than 145 nm..

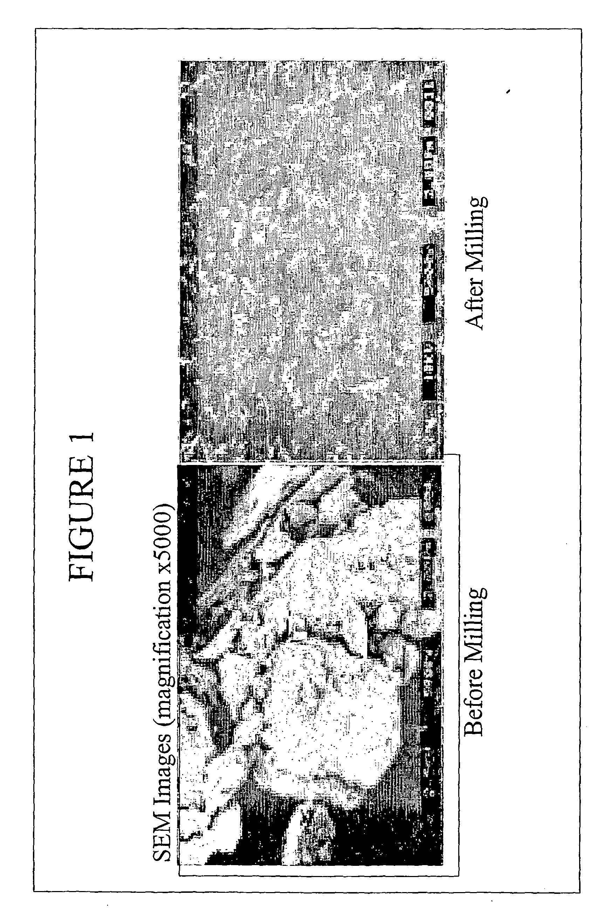

FIG. 1 shows SEM images of insulin particles before and after milling, clearly demonstrating the effect of the milling and, more importantly, the capacity of the surface stabilizers to prevent agglomeration of the insulin particles. Analysis of a sample of this material stored for six months at 5°C revealed 90% of the insulin particles to be smaller than 145 nm.

### Example 4

The purpose of this example was to determine the insulin content of a nanoparticulate composition following milling, and to determine if the milling process results in loss of insulin.

Nanoparticulate insulin was prepared in accordance with the procedure of Example 1 from a slurry of 2% insulin having 1 % Pluronic® F68 and 0.1 % sodium deoxycholic acid as surface stabilizers.

Aliquots of the nanoparticulate insulin were centrifuged at 13,000 g for 15 min. The supernatant was isolated and the pellet washed in Phosphate Buffer Saline (PBS) and re-spun. The pellets and supernatant fractions post-centrifugation were subjected to analysis on an SDS-PAGE gel. Raw insulin in PBS additionally containing 0.1 M HCl for stability (Sigma I-5500) served as a control.

Reducing gels used in the analysis were prepared as 16% acrylamide gels and comprised 5.33 ml of acrylamide solution, 2.02 ml distilled water, 2.50 ml of 1.5 M TRIS buffer at pH of 8.8, 100 µm 10% SDS, 50 µm 10% ammonium persulfate, and 5 µm of TEMED (N,N,N',N'-tetramethylethylene diamine). Reducing gels are useful for visualizing insulin bands, which appear as a dimer, tetramer, etc., and for identifying degraded insulin fragments.

The composition of the running buffer and loading buffers were as follows:

| **5X Electrode Buffer** | | **Sample Loading Buffer** | |
|---|---|---|---|
| (1X = 25 mM Tris, 192 mM Glycine, 0.1% SDS, pH 8.3) | | (SDS reducing buffer: 62.5 mM Tris HCl (pH6.8), 20% Glycerol, 2% SDS, 5% β-mercaptoethanol) | |
| Tris Base | 37.5g | | |
| Glycine | 180.0g | Distilled water | 3.0 ml |
| SDS | 12.5g | 0.5M Tris-HCl (pH6.8) | 1.0 ml |
| Distilled Water | 2.5L | Glycerol | 1.6 ml |
| | | 10% SDS | 1.6 ml |
| Dilute 200 ml of the 5X stock with 800 ml distilled water before use. | | β-mercaptoethanol | 0.4 ml |
| | | 0.5% (w/v) bromophenol blue 0.4 ml (in water) | |

The samples and the supernatant were diluted to 1:10 and 1:50, respectively, with sample buffer and then heated to 95°C for 5 mins before loading onto the gel. The volume of supernatant loaded was the amount that would be needed to load the required amount of sample, if the insulin were left in suspension.

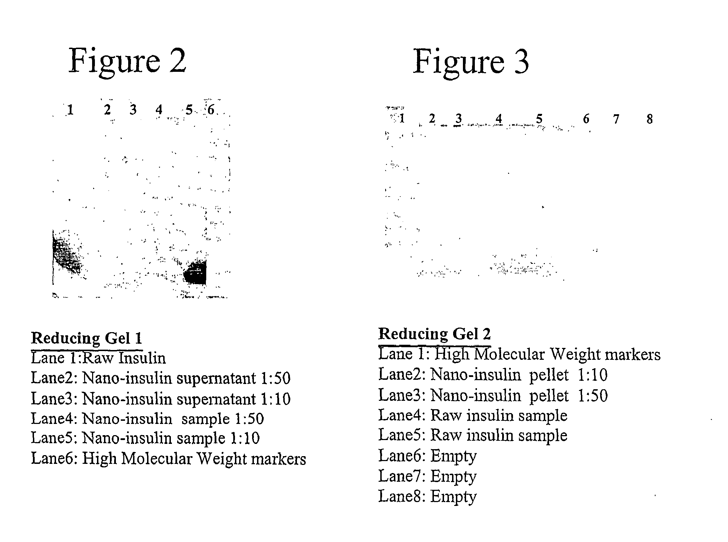

Two reducing gels were prepared, and the samples were loaded onto the gels so that the pellet and supernatant fractions from each sample could be observed side by side. The results are shown in FIG. 2 and FIG. 3.
The reducing gel shown in FIG. 2 was loaded with the following:
- Lane 1:: Raw Insulin Sample
- Lane 2:: Sample supernatant 1:50 dilution;
- Lane 3:: Sample supernatant 1:10 dilution;
- Lane 4:: Pellet Sample 1:50 dilution;
- Lane 5:: Pellet Sample 1:10 dilution; and
- Lane 6:: High Molecular Weight markers.
The reducing gel shown in FIG. 3 was loaded with the following:
- Lane 1:: High Molecular Weight markers;
- Lane 2:: Pellet Sample 1:10 dilution;
- Lane 3:: Pellet Sample 1:50 dilution;
- Lane 4:: Raw Insulin Sample; and
- Lane 5:: Raw Insulin Sample.

The gels shown in FIGs 2 and 3 demonstrate that the pellet fraction of the milled insulin formulation contains all of the insulin. The lanes loaded with supernatant appear to be free of insulin. This indicates that all of the insulin in the nanoparticulate insulin composition is entrapped within or associated with the nanoparticulate composition, with little or no insulin free in solution. Thus, there is essentially 100% entrapment or association of the insulin within the nanoparticulate composition.

### Example 5

The purpose of this example was to test the effectiveness of nanoparticulate insulin compositions parenterally administered, and to compare the effectiveness of the formulations with conventional raw insulin compositions.

All animals (Wistar rats) were weighed and fasted for 4 hours prior to study initiation. The rats (250-300 g) were anaesthetized by intra-muscular (IM) administration of a solution of ketamine (22.5 mg/animal) and acepromazine (0.75 mg/kg) one hour prior to administration of the test solutions. The sample of nanoparticulate insulin prepared in accordance with the procedure of Example 1 from a slurry of 2% insulin having 1% Pluronic® F68 and 0.1% sodium deoxycholic acid as surface stabilizers was formulated as a solution in PBS (phosphate buffer solution) pH 7.4. Groups of 6 animals received an amount of the solution sufficient to provide one IU of insulin by subcutaneous, intramuscular, or intraperitoneal injection in a randomized non-crossover study. Blood glucose values for the rats were measured using an Accutren® alpha glucometer (Boehringer Mannhein) following systemic blood samples that were taken from the tail artery. A baseline blood sample was taken and samples were taken at intervals post injection up to four hours. Anesthesia was maintained throughout the study. A non-formulated insulin solution was utilized as a reference.

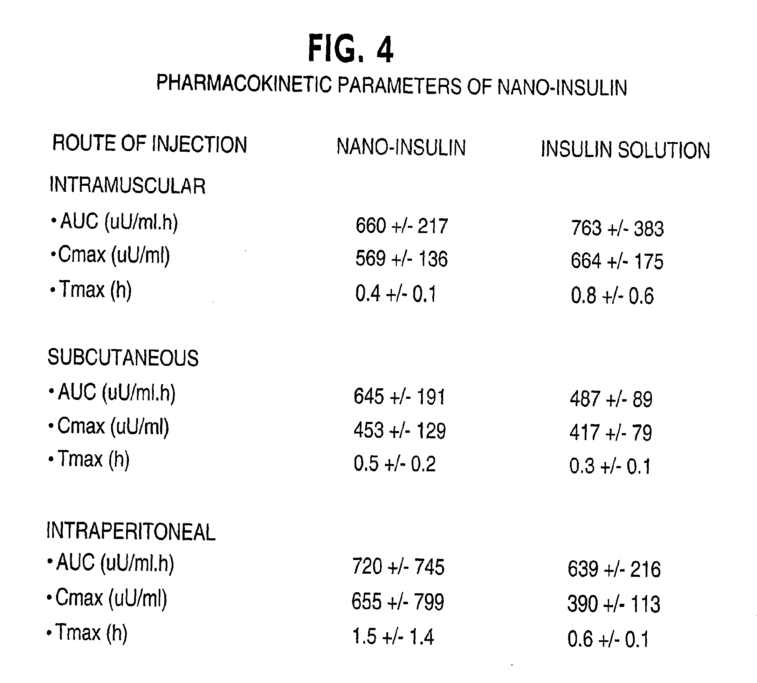

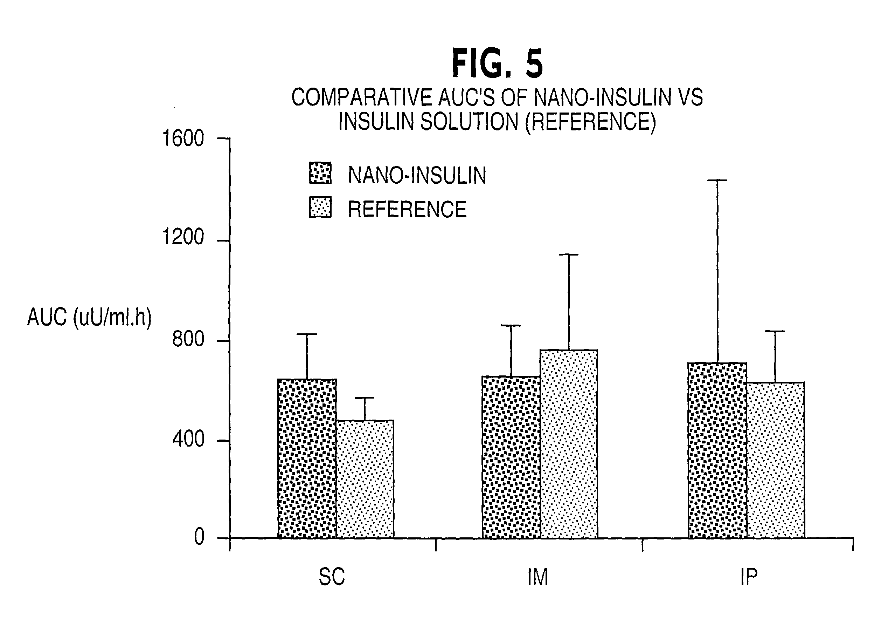

The rat model used is a hypergylcemic model. The pharmacokinetic parameters of the test are given in FIG. 4. It can be seen from the results in FIG. 4 that the insulin compositions of the present invention are biologically active and at least approximately equal to the reference insulin solution. Fig. 5 illustrates the AUC (*µ*U/ml.h) for the sample by the three modes of parenteral administration in comparison to the reference insulin solution. The analysis shown in FIG. 5 likewise establishes the equivalence of the test preparation to the reference insulin solution.

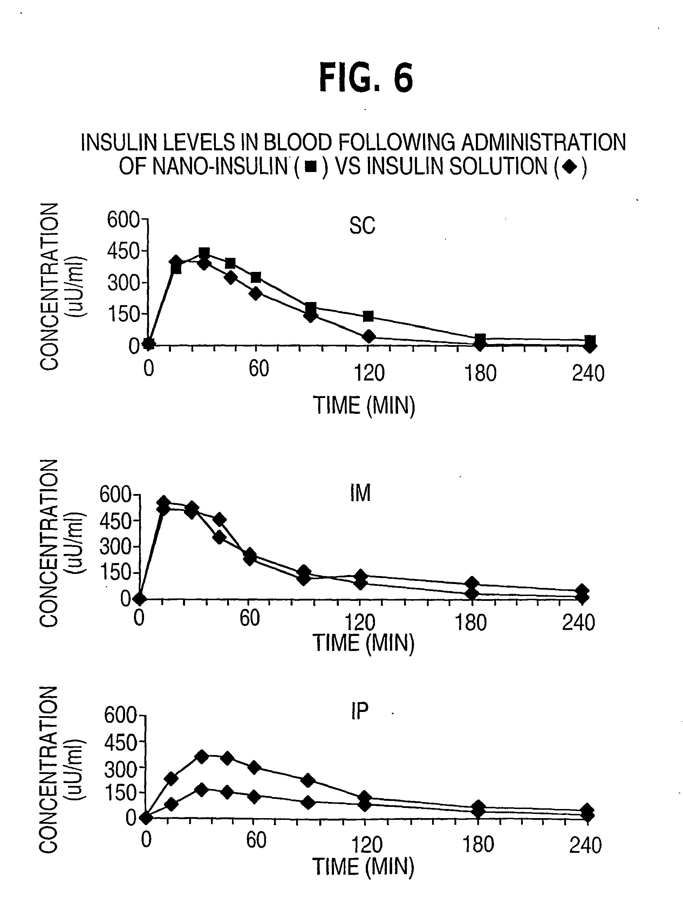

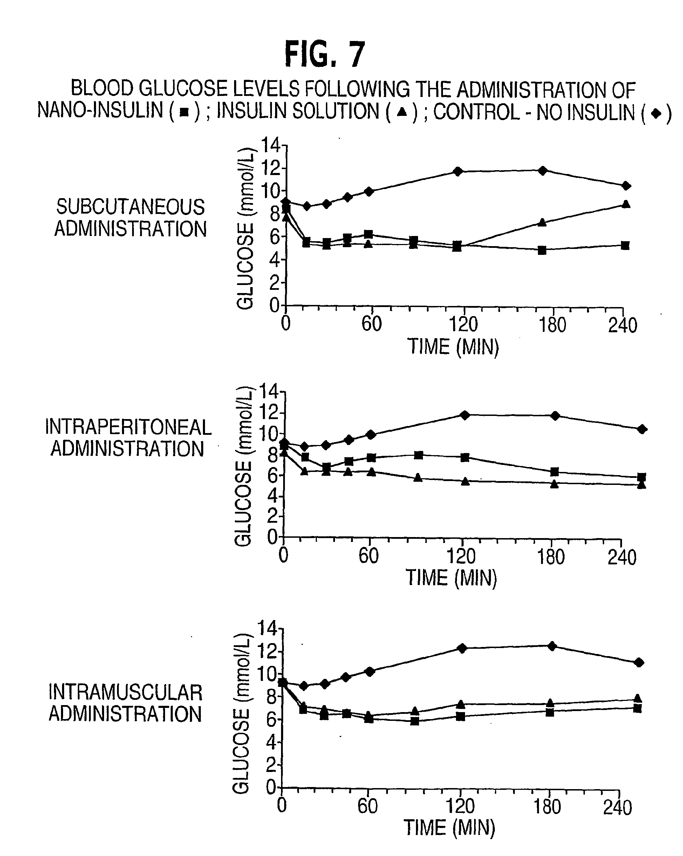

FIGs 6 and 7 are companion concentration-time profiles in that FIG 6 illustrates mean glucose concentration while FIG. 7 illustrates mean insulin concentration. As described above, both plot the test sample administered via subcutaneous, intramuscular and intraperitoneal injection in comparison to a reference insulin preparation. If an insulin preparation is biologically active, a fall in blood glucose levels is expected to coincide with the rise in blood insulin levels. The profiles in FIGs 6 and 7 clearly establish that the nanoparticulate insulin formulations of the invention are biologically active.

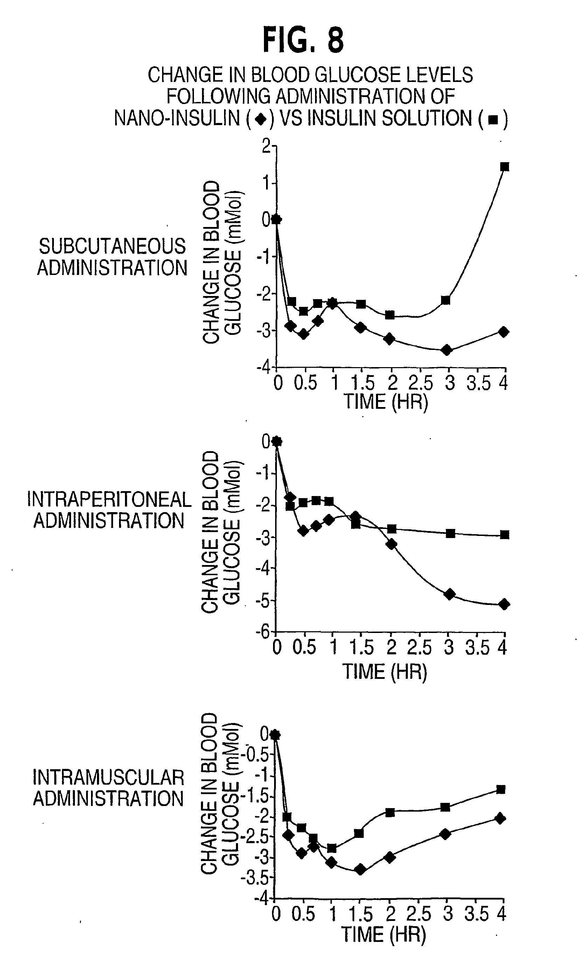

Overall, the results reported in the figures demonstrate that the nanoparticulate insulin compositions of the present invention have excellent bioactivity following parenteral administration, which is at least equivalent to the reference insulin solution tested. In the plot of change in blood glucose level plotted in FIG. 8, it can be seen that the nanoparticle preparation of the present invention is advantageous over the reference insulin solution in maintaining a lowering of the blood glucose level over the duration of the test. A particular advantage can be seen from the comparison via intraperitoneal administration.

## Claims

1. A nanoparticulate composition comprising insulin particles and at least one non-crosslinked surface stabilizer adsorbed on the surface thereof, wherein the insulin particles have an effective average particle size of less than about 5 microns.

2. The composition of claim 1, wherein the effective average particle size of the insulin particles is selected from the group consisting of less than about 4 microns, less than about 3 microns, less than about 2 microns, less than about 1500 nm, less than about 1 micron, less than about 800 nm, less than about 700 nm, less than about 600 nm, less than about 500 nm, less than about 400 nm, less than about 300 nm, less than about 200 nm, less than about 100 nm, less than about 75 nm, and less than about 50 nm.

3. The composition of claims 1 or 2, wherein the concentration of insulin and/or the at least one surface stabilizer is selected from the group consisting of from about 99.5% to about 0.001 %, from about 95% to about 0.1%, and from about 90% to about 0.5%, by weight, based on the total combined dry weight of the insulin particles and at least one surface stabilizer, not including other excipients.

4. The composition according to any one of claims 1 to 3, wherein the composition further comprises one or more pharmaceutically acceptable excipients, carriers, or a combination thereof.

5. The composition according to any one of claims 1 to 4, wherein the composition is formulated for administration selected from the group consisting of oral, pulmonary, nasal, parenteral, rectal, local, buccal, and topical administration.

6. The composition according to any one of claims 1 to 5, wherein the insulin is in a form selected from the group consisting of crystalline particles, semi-crystalline particles, semi-amorphous particles, amorphous particles, and a mixture thereof.

7. The composition according to any one of claims 1 to 6, further comprising at least two surface stabilizers.

8. The composition according to any one of claims 1 to 7, wherein the at least one surface stabilizer is selected from the group consisting of a nonionic surface stabilizer, an anionic surface stabilizer, a cationic surface stabilizer, and an ionic surface stabilizer.

9. The composition according to any one of claims 1 to 7, wherein the at least one surface stabilizer is selected from the group consisting of cetyl pyridinium chloride, gelatin, casein, phosphatides, dextran, glycerol, gum acacia, cholesterol, tragacanth, stearic acid, stearic acid esters and salts, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylmethyl-cellulose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers, poloxamines, a charged phospholipid, dimyristoyl phophatidyl glycerol, dioctylsulfosuccinate, dialkylesters of sodium sulfosuccinic acid, sodium lauryl sulfate, alkyl aryl polyether sulfonates, mixtures of sucrose stearate and sucrose distearate, triblock copolymers of the structure: -(-PEO)-(-PBO-)--(-PEO-)-, p-isononylphenoxypoly-(glycidol), decanoyl-N-methylglucamide; n-decyl β-D-glucopyranoside, n-decyl β-D-maltopyranoside, n-dodecyl β-D-glucopyranoside, n-dodecyl β-D-maltoside, heptanoyl-N-methylglucamide, n-heptyl-β-D-glucopyranoside, n-heptyl β-D-thioglucoside, n-hexyl β-D-glucopyranoside, nonanoyl-N-methylglucamide, n-noyl β-D-glucopyranoside, octanoyl-N-methylglucamide, n-octyl-β-D-glucopyranoside, octyl β-D-thioglucopyranoside, lysozyme, a PEG derivatized phospholipid, PEG derivatized cholesterol, a PEG derivatized cholesterol derivative, PEG derivatized vitamin A, PEG derivatized vitamin E, and random copolymers of vinyl acetate, vinyl pyrrolidone, cationic lipids, benzalkonium chloride, sulfonium compounds, phosphonium compounds, quarternary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10^{™}, tetrabutylammonium bromide, benzyl trimethylammonium bromide, choline esters, benzalkonium chloride, stearalkonium chloride compounds, cetyl pyridinium bromide, cetyl pyridinium chloride, halide salts of quatemized polyoxyethylalkylamines, MIRAPOL^{™}, ALKAQUAT^{™}, alkyl pyridinium salts; amines, amine salts, amine oxides, imide azolinium salts, protonated quaternary acrylamides, methylated quaternary polymers, cationic guar, polymethylmethacrylate trimethylammonium bromide, polyvinylpyrrolidone-2-dimethylaminoethyl methacrylate dimethyl sulfate, hexadecyltrimethyl ammonium bromide, poly (2-methacryloxyethyltrimethylammonium bromide) (S1001), poly(N-vinylpyrrolidone/2-dimethylaminoethyl methacrylate) di methylsulphate quarternary (S1002), and poly(2-methylacryloxyamidopropyltrimethylammonium chloride) (S1004).

10. The composition of claim 8, wherein the cationic surface stabilizer is selected from the group consisting of a polymer, a biopolymer, a polysaccharide, a cellulosic, an alginate, a nonpolymeric compound, and a phospholipid.

11. The composition according to any one of claims 1 to 10 for use in therapy.

12. A dosage form comprising the nanoparticulate insulin composition according to any one of claims 1 to 11, wherein the dosage form is a solid, semi-solid, or liquid dosage formulation.

13. A dosage form comprising the nanoparticulate insulin composition according to any one of claims 1 to 11, wherein the dosage form is selected from the group consisting of controlled release formulations, solid dose fast melt formulations, aerosol formulations, lyophilized formulations, tablets, solid lozenge, capsules, powders, and liquids for injection.

14. A pharmaceutical composition for parenteral administration comprising the nanoparticulate insulin composition according to any one of claims 1 to 11, or the dosage form of claim 12 or 13, and a suitable, pharmaceutically acceptable excipient.

15. A method of making the nanoparticulate insulin composition according to any one of claims 1 to 11 comprising contacting insulin particles with at least one surface stabilizer for a time and under conditions sufficient to provide a nanoparticulate insulin composition having an effective average particle size of less than about 5 microns.

16. The method of claim 15, wherein said contacting comprises milling.

17. The method of claim 15, wherein said contacting comprising homogenizing.

18. The method of claim 16, wherein said milling is high energy milling or wet milling.

19. Use of the nanoparticulate insulin composition according to any one of claims 1 to 11 for the preparation of a pharmaceutical composition for insulin therapy.

## Patentansprüche

1. Eine nanopartikuläre Zusammensetzung umfassend Insulinpartikel und mindestens einen nicht-quervemetzen Oberflächenstabilisator, der an ihrer Oberfläche adsorbiert ist, wobei die Insulinpartikel eine effektive durchschnittliche Partikelgröße von weniger als circa 5 Mikron haben.

2. Die Zusammensetzung gemäß Anspruch 1, wobei die effektive durchschnittliche Partikelgröße der Insulinpartikel ausgewählt ist aus der Gruppe bestehend aus weniger als circa 4 Mikron, weniger als circa 3 Mikron, weniger als circa 2 Mikron, weniger als circa 1500 nm, weniger als circa 1 Mikron, weniger als circa 800 nm, weniger als circa 700 nm, weniger als circa 600 nm, weniger als circa 500 nm, weniger als circa 400 nm, weniger als circa 300 nm, weniger als circa 200 nm, weniger als circa 100 nm, weniger als circa 75 nm und weniger als circa 50 nm.

3. Die Zusammensetzung gemäß Anspruch 1 oder 2, wobei die Konzentration des Insulins und/oder des mindestens einen Oberflächenstabilisators ausgewählt ist aus der Gruppe bestehend aus von etwa 99,5% bis etwa 0,001%, von etwa 95% bis etwa 0,1 % und von etwa 90% bis etwa 0,5% vom Gewicht, ausgehend vom gesamten kombinierten Trockengewicht der Insulinpartikel und des mindestens eines Oberflächenstabilisators, nicht umfassend andere Hilfsstoffe.

4. Die Zusammensetzung gemäß einem der Ansprüche 1 bis 3, wobei die Zusammensetzung weiterhin einen oder mehrere pharmazeutisch zulässige Hilfsstoffe, Träger, oder eine Kombination davon umfaßt.

5. Die Zusammensetzung gemäß einem der Ansprüche 1 bis 4, wobei die Zusammensetzung zur Verabreichung ausgewählt aus der Gruppe, bestehend aus oraler, pulmonaler, nasaler, parenteraler, rektaler, lokaler, bukkaler und topischer Verabreichungen, formuliert ist.

6. Die Zusammensetzung gemäß einer der Ansprüche 1 bis 5, wobei das Insulin in einer Form ausgewählt aus der Gruppe bestehend aus kristallinen Partikeln, semikristallinen Partikeln, semi-amorphen Partikeln, amorphen Partikeln, und einer Mischung davon, vorliegt.

7. Die Zusammensetzung gemäß einer der Ansprüche 1 bis 6, weiterhin umfassend mindestens zwei Oberflächenstabilisatoren.

8. Die Zusammensetzung gemäß einer der Ansprüche 1 bis 7, wobei der mindestens eine Oberflächenstabilisator ausgewählt ist aus der Gruppe bestehend aus einem nichtionischen Oberflächenstabilisator, einem anionischen Oberflächenstabilisator, einem kationischen Oberflächenstabilisator und einem ionischen Oberflächenstabilisator.

9. Die Zusammensetzung gemäß einer der Ansprüche 1 bis 7, wobei der mindestens eine Oberflächenstabilisator ausgewählt ist aus der Gruppe bestehend aus Cetylpyridiniumchlorid, Gelatine, Casein, Phosphatide, Dextran, Glycerin, Akaziengummi, Cholesterin, Tragant, Stearinsäure, Stearinsäureester und Salze, Calciumstearat, Glycerinmonostearate, Cetylstearylalkohol, Cetylmacrogol Emulsifying Wax, Sorbitanester, Polyoxyethylenalkylether, Polyoxyethylenricinusöl-Derivate, Polyoxyethylensorbitanfettsäureester, Polyethylenglykol, Dodecyltrimethylammoniumbromid, Polyoxyethylenstearate, kolloidales Siliciumdioxid, Phosphate, Natriumdodecylsulfat, Calciumcarboxymethylcellulose, Hydroxypropylcellulose, Hydroxypropylmethylcellulose, Carboxymethylcellulose, Methylcellulose, Hydroxyethylcellulose, Hydroxypropylmethylcellulose-Phthalsäureester, nichtkristalline Cellulose, Magnesiumaluminiumsilicat, Triethanolamin, Polyvinylalcohol, Polyvinylpyrrolidon, 4-(1,1,3,3-tetramethylbutyl)-phenol Polymer mit Ethylenoxid und Formaldehyd, Poloxamere, Poloxamine, ein geladenes Phospholipid, Dimyristoylphophatidylglycerin, Dioctylsulfosuccinat, dialkylester der Natriumsulfobernsteinsäure, Natriumlaurylsulfat, Alkylarylpolyether sulfonate, Mischungen von Saccharosestearat und Saccharosedistearat, Triblock-copolymere der Struktur: -(-PEO)--(-PBO-)--(-PEO-)-, p-Isononylphenoxypoly-(glycidol), Decanoyl-N-methylglucamid; n-Decyl-β-D-glucopyranosid, n-Decyl-β-D-maltopyranosid, n-Dodecyl-β-D-glucopyranosid, n-Dodecyl-β-D-maltosid, Heptanoyl-N-methylglucamid, n-Heptyl-β-D-glucopyranosid, n-Heptyl-β-D-thioglucosid, n-Hexyl-β-D-glucopyranosid, Nonanoyl-N-methylglucamid, n-Noyl-β-D-glucopyranosid, Octanoyl-N-methylglucamid, n-Octyl-β-D-glucopyranosid, Octyl-β-D-thioglucopyranosid, Lysozym, ein PEG derivatisiertes Phospholipid, PEG derivatisiertes Cholesterin, ein PEG derivatisiertes Cholesterinderivat, PEG derivatisiertes Vitamin A, PEG derivatisiertes Vitamin E, und Random-Copolymere von Vinylacetat, Vinylpyrrolidon, kationische Lipide, Benzalkoniumchlorid, Sulfoniumverbindungen, Phosphoniumverbindungen, quartäre Ammoniumverbindungen, Benzyl-di(2-chloroethyl)ethylammoniumbromid, Cocosnußtrimethylammoniumchlorid, Cocosnußtrimethylammoniumbromid, Cocosnußmethyldihydroxyethylammoniumchlorid, Cocosnußmethyldihydroxyethylammoniumbromid, Decyltriethylammoniumchlorid, Decyldimethylhydroxyethylammoniumchlorid, Decyldimethylhydroxyethylammoniumchloridbromid, C₁₂₋₁₅dimethyl hydroxyethylammoniumchlorid, C₁₂₋₁₅ dimethylhydroxyethylammoniumchloridbromid, Cocosnußdimethylhydroxyethylammoniumchlorid, Cocosnußdimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethenoxy)₄ammoniumchlorid, Lauryldimethyl (ethenoxy)₄ammoniumbromid, N-Alkyl (C₁₂₋₁₈)dimethylbenzylammoniumchlorid, N-Alkyl (C₁₄₋₁₈)dimethyl-benzylammoniumchlorid, N-Tetradecylidmethylbenzylammoniumchloridmonohydrat, Dimethyldidecylammoniumchlorid, N-Alkyl- und (C₁₂₋₁₄)-dimethyl-1-napthylmethylammoniumchlorid, Trimethylammoniumhalid, Alkyltrimethylammoniumsalze, Dialkyl-dimethylammoniumsalze, Lauryltrimethylammoniumchlorid, ethoxyliertes Alkyamidoalkyldialkylammoniumsalz, ein ethoxyliertes Trialkylammoniumsalz, Dialkylbenzoldialkylammoniumchlorid, N-Didecyldimethylammoniumchlorid, N-Tetradecyldimethylbenzylammoniumchlorid-monohydrat, N-Alkyl(C₁₂₋₁₄)-dimethyl-1-naphthylmethylammoniumchlorid, Dodecyldimethylbenzylammonium chlorid, Dialkylbenzolealkylammoniumchlorid, Lauryltrimethylammoniumchlorid, Alkylbenzylmethylammoniumchlorid, Alkylbenzyldimethylammoniumbromid, C₁₂Trimethylammoniumbromide, C₁₅Trimethylammoniumbromide, C₁₇Trimethylammoniumbromide, Dodecylbenzyltriethylammoniumchlorid, Poly-diallyldimethylammoniumchlorid (DADMAC), Dimethylammoniumchloride, Alkyldimethylammoniumhalogenide, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT 10^{™}, Tetrabutylammoniumbromid, Benzyltrimethylammoniumbromid, Cholinester, Benzalkoniumchlorid, Stearalkoniumchlorid Verbindungen, Cetylpyridiniumbromid, Cetylpyridiniumchlorid, Halide von quatämizierten Polyoxyethylalkylaminen, MIRAPOL^{™}, ALKAQUAT^{™}, Alkylpyridiniumsalze; Amine, Aminsalze, Aminoxide, Imide-azoliniumsalze, protonierte quartäre Acrylamide, methylierte quartäre Polymere, kationischer Guar, Polymethylmethacrylat-trimethylammoniumbromid, Polyvinylpyrrolidon-2-dimethylaminoethylmethacrylatdimethylsulfat, Hexadecyltrimethylammoniumbromid, poly(2-Methacryloxyethyltrimethylammoniumbromid) (S1001), quartäres poly(N-Vinylpyrrolidon/2-dimethylaminoethylmethacrylat)-di-methylsulfat (S 1002), und poly(2-Methylacryloxyamidopropyltrimethylammoniumchlorid) (S 1004).

10. Die Zusammensetzung gemäß Anspruch 8, wobei der kationische Oberflächenstabilisator ausgewählt ist aus der Gruppe bestehend aus einem Polymer, einem Biopolymer, einem Polysaccharid, einer Cellulose, einem Alginat, einer nicht-polymeren Verbindung, und einem Phospholipid.

11. Die Zusammensetzung gemäß einer der Ansprüche 1 bis 10 zur Verwendung in der Therapie.

12. Eine Dosierungsform umfassend die nanopartikuläre Insulinzusammensetzung gemäß einer der Ansprüche 1 bis 11, wobei die Dosierungsform eine feste, halbfeste oder flüssige Dosierungsformulierung ist.

13. Eine Dosierungsform umfassend die nanopartikuläre Insulinzusammensetzung gemäß einer der Ansprüche 1 bis 11, wobei die Dosierungsform ausgewählt ist aus der Gruppe bestehend aus Formulierungen für die kontrollierte Freisetzung, schnellschmelzenden festen Dosierungsformulierungen, Aerosolformulierungen, lyophilisierten Formulierungen, Tabletten, festen Lutschtabletten, Kapseln, Pulvern und Flüssigkeiten zur Injektion.

14. Eine pharmazeutische Zusammensetzung zur parenteralen Verabreichung umfassend die nanopartikuläre Insulinzusammensetzung gemäß einer der Ansprüche 1 bis 11 oder die Dosierungsform der Ansprüche 12 oder 13 und einen geeigneten, pharmazeutisch zulässigen Hilfsstoff.

15. Ein Verfahren zur Herstellung der nanopartikuläre Insulinzusammensetzung gemäß einer der Ansprüche 1 bis 11 umfassend das In-Kontakt-Bringen der Insulinpartikel mit mindestens einem Oberflächenstabilisator für eine Zeit und unter Bedingungen die ausreichend sind um eine nanopartikuläre Insulinzusammensetzung mit einer effektiven durchschnittlichen Partikelgröße von weniger als circa 5 Mikron bereitzustellen.

16. Das Verfahren gemäß Anspruch 15, wobei das genannte In-Kontakt-Bringen das Mahlen umfaßt.

17. Das Verfahren gemäß Anspruch 15, wobei das genannte In-Kontakt-Bringen die Homogenisierung umfaßt.

18. Das Verfahren gemäß Anspruch 16, wobei das genannte Mahlen das Hochenergiemahlen und das Nassmahlen umfaßt.

19. Verwendung einer nanopartikulären Insulinformulierung gemäß einem der Ansprüche 1 bis 11 zur Herstellung einer pharmazeutischen Zusammensetzung zur Insulintherapie.

## Revendications

1. Composition nanoparticulaire comprenant des particules d'insuline et au moins un stabilisant de surface non réticulé adsorbé sur leur surface, dans laquelle les particules d'insuline ont une taille de particule moyenne effective de moins d'environ 5 microns.

2. Composition selon la revendication 1, dans laquelle la taille de particule moyenne effective des particules d'insuline est choisie dans le groupe constitué par moins d'environ 4 microns, moins d'environ 3 microns, moins d'environ 2 microns, moins d'environ 1500 nm, moins d'environ 1 micron, moins d'environ 800 nm, moins d'environ 700 nm, moins d'environ 600 nm, moins d'environ 500 nm, moins d'environ 400 nm, moins d'environ 300 nm, moins d'environ 200 nm, moins d'environ 100 nm, moins d'environ 75 nm, et moins d'environ 50 nm.

3. Composition selon la revendication 1 ou 2, dans laquelle la concentration d'insuline et/ou de l'au moins un stabilisant de surface est choisie dans le groupe constitué par d'environ 99,5 % à environ 0,001 %, d'environ 95 % à environ 0,1 %, et d'environ 90 % à environ 0,5 %, en poids, par rapport au poids sec combiné total des particules d'insuline et d'au moins un stabilisant de surface, sans inclure d'autres excipients.

4. Composition selon l'une quelconque des revendications 1 à 3, la composition comprenant en outre un ou plusieurs excipients, véhicules pharmaceutiquement acceptables, ou une combinaison de ceux-ci.

5. Composition selon l'une quelconque des revendications 1 à 4, la composition étant formulée pour une administration choisie dans le groupe constitué par l'administration orale, pulmonaire, nasale, parentérale, rectale, locale, buccale et topique.

6. Composition selon l'une quelconque des revendications 1 à 5, dans laquelle l'insuline est sous une forme choisie dans le groupe constitué par les particules cristallines, les particules semi-cristallines, les particules semi-amorphes, les particules amorphes, et un mélange de celles-ci.

7. Composition selon l'une quelconque des revendications 1 à 6, comprenant en outre au moins deux stabilisants de surface.

8. Composition selon l'une quelconque des revendications 1 à 7, dans laquelle l'au moins un stabilisant de surface est choisi dans le groupe constitué par un stabilisant de surface non ionique, un stabilisant de surface anionique, un stabilisant de surface cationique et un stabilisant de surface ionique.

9. Composition selon l'une quelconque des revendications 1 à 7, dans laquelle l'au moins un stabilisant de surface est choisi dans le groupe constitué par le chlorure de cétylpyridinium, la gélatine, la caséine, les phosphatides, le dextrane, le glycérol, la gomme arabique, le cholestérol, la gomme adragante, l'acide stéarique, les esters et les sels de l'acide stéarique, le stéarate de calcium, le monostéarate de glycérol, l'alcool cétostéarylique, la cire émulsifiante Cetomacrogol, les esters de sorbitanne, les éthers alkyliques de polyoxyéthylène, les dérivés polyoxyéthyléniques d'huile de ricin, les esters polyoxyéthyléniques d'acides gras de sorbitanne, les polyéthylèneglycols, le bromure de dodécyltriméthylammonium, les stéarates de polyoxyéthylène, le dioxyde de silicium colloïdal, les phosphates, le dodécylsulfate de sodium, la carboxyméthylcellulose calcique, les hydroxypropylcelluloses, l'hydroxypropyl-méthylcellulose, la carboxyméthylcellulose sodique, la méthylcellulose, l'hydroxyéthylcellulose, le phtalate d'hydroxypropylméthylcellulose, la cellulose non cristal-line, le silicate de magnésium et d'aluminium, la triéthanolamine, l'alcool polyvinylique, la polyvinyl-pyrrolidone, le polymère 4-(1,1,3,3-tétraméthylbutyl)-phénolique avec l'oxyde d'éthylène et le formaldéhyde, les poloxamères, les poloxamines, un phospholipide chargé, le dimyristoyl-phosphatidyl-glycérol, le dioctylsulfosuccinate, les dialkylsulfosuccinates de sodium, le laurylsulfate de sodium, les polyéthersulfonates d'alkylaryle, les mélanges de stéarate de saccharose et de distéarate de saccharose, les copolymères triséquencés de structure : -(-PEO)-(-PBO-)--(-PEO-)-, le p-isononylphénoxypoly-(glycidol), le décanoyl-N-méthyl-glucamide ; le n-décyl-β-D-glucopyranoside, le n-décyl-β-D-maltopyranoside, le n-dodécyl-β-D-glucopyranoside, le n-dodécyl-β-D-maltoside, l'heptanoyl-N-méthylglucamide, le n-heptyl-β-D-glucopyranoside, le n-heptyl-β-D-thioglucoside, le n-hexyl-β-D-glucopyranoside, le nonanoyl-N-méthyl-glucamide, le n-nonyl-β-D-glucopyranoside, l'octanoyl-N-méthylglucamide, le n-octyl-β-D-glucopyranoside, l'octyl-β-D-thioglucopyranoside, le lysozyme, un phospholipide transformé en dérivé avec du PEG, le cholestérol transformé en dérivé avec du PEG, un dérivé du cholestérol transformé en dérivé avec du PEG, la vitamine A transformée en dérivé avec du PEG, la vitamine E transformée en dérivé avec du PEG, et les copolymères statistiques d'acétate de vinyle, la vinylpyrrolidone, les lipides cationiques, le chlorure de benzalkonium, les composés du sulfonium, les composés du phosphonium, les composés d'ammonium quaternaire, le bromure de benzyl-di(2-chloréthyl)éthylammonium, le chlorure de triméthylammonium dérivé du coprah, le bromure de triméthylammonium dérivé du coprah, le chlorure de méthyldihydroxyéthylammonium dérivé du coprah, le bromure de méthyldihydroxyéthylammonium dérivé du coprah, le chlorure de décyltriéthylammonium, le chlorure de décyldiméthylhydroxyéthylammonium, le bromure-chlorure de décyldiméthylhydroxyéthylammonium, le chlorure de (C₁₂-C₁₅)-diméthylhydroxyéthylammoniumm, le bromure-chlorure de (C₁₂-C₁₅)-diméthylhydroxyéthylammonium, le chlorure de diméthylhydroxyéthylammonium dérivé du coprah, le bromure de diméthylhydroxyéthylammonium dérivé du coprah, le méthylsulfate de myristyltriméthylammonium, le chlorure de lauryldiméthylbenzylammonium, le bromure de lauryldiméthyl-benzylammonium, le chlorure de lauryldiméthyl-(éthénoxy)₄-ammonium, le bromure de lauryldiméthyl-(éthénoxy)₄-ammonium, le chlorure de N-alkyl-(C₁₂₋₁₈)diméthylbenzylammonium, le chlorure de N-alkyl-(C₁₄₋₁₈)diméthylbenzylammonium, le monohydrate de chlorure de N-tétradécyldiméthylbenzyl-ammonium, le chlorure de diméthyldidécylammonium, le chlorure de N-alkyl- et (C₁₂₋₁₄)-diméthyl-1-naphtylméthylammonium, l'halogénure de triméthylammonium, les sels d'alkyl-triméthylammonium, les sels de dialkyldiméthylammonium, le chlorure de lauryltriméthyl-ammonium, le sel d'alkylamidoalkyldialkylammonium éthoxylé, un sel de trialkylammonium éthoxylé, le chlorure de dialkylbenzène-dialkylammonium, le chlorure de N-didécyldiméthylammonium, le monohydrate de chlorure de N-tétradécyldiméthylbenzylammonium, le chlorure de N-alkyl-(C₁₂₋₁₄) diméthyl-1-naphtylméthylammonium, le chlorure de dodé-cyldiméthylbenzylammonium, le chlorure de dialkylbenzène-alkylammonium, le chlorure de lauryltriméthylammonium, le chlorure d'alkylbenzylméthylammonium, le bromure d'alkylbenzyldiméthylammonium, les bromures de C₁₂-tri-méthylammonium, les bromures de C₁₅-triméthylammonium, les bromures de C₁₇-triméthylammonium, le chlorure de dodécylbenzyltriéthylammonium, le chlorure de polydiallyldiméthylammonium (DADMAC), les chlorures de diméthylammonium, les halogénures d'alkyldiméthylammonium, le chlorure de tricétylméthylammonium, le bromure de décyltriméthylammonium, le bromure de dodécyltriéthyl-ammonium, le bromure de tétradécyltriméthylammonium, le chlorure de méthyltrioctylammonium, le POLYQUAT 10^{™}, le bromure de tétrabutylammonium, le bromure de benzyltriméthylammonium, les esters de choline, le chlorure de benzalkonium, les composés du chlorure de stéaralkonium, le bromure de cétylpyridinium, le chlorure de cétylpyridinium, les sels halogénures de polyoxyéthylalkylamines quaternisées, le MIRAPOL^{™}, l'ALKAQUAT^{™}, les sels d'alkylpyridinium ; les amines, les sels d'amines, les oxydes d'amines, les sels d'imidazolinium, les acrylamides quaternaires protonés, les polymères quaternaires méthylés, le guar cationique, le bromure de polyméthylméthacrylate-triméthylammonium, le diméthylsulfate de polyvinylpyrrolidone-2-diméthylamino-éthylméthacrylate, le bromure d'hexadécyltriméthylammonium, le poly(bromure de 2-méthacryloxyéthyltriméthylammonium) (S1001), le diméthylsulfate quaternaire de poly(N-vinyl-pyrrolidone/2-diméthylaminoéthyl-méthacrylate) (S1002) et le poly(chlorure de 2-méthylacryloxyamidopropyltri-méthylammonium) (S 1004).

10. Composition selon la revendication 8, dans laquelle le stabilisant de surface cationique est choisi dans le groupe constitué par un polymère, un biopolymère, un polysaccharide, un dérivé de cellulose, un alginate, un composé non polymère et un phospholipide.

11. Composition selon l'une quelconque des revendications 1 à 10, pour utilisation en thérapie.

12. Forme galénique comprenant la composition d'insuline nanoparticulaire selon l'une quelconque des revendications 1 à 11, la forme galénique étant une formulation galénique solide, semi-solide ou liquide.

13. Forme galénique comprenant la composition d'insuline nanoparticulaire selon l'une quelconque des revendications 1 à 11, la forme galénique étant choisie dans le groupe constitué par les formulations à libération contrôlée, les formulations en doses solides fondant rapidement, les formulations en aérosol, les formulations lyophilisées, les comprimés, les pastilles pleines, les capsules, les poudres et les liquides pour injection.

14. Composition pharmaceutique pour l'administration parentérale comprenant la composition d'insuline nanoparticulaire selon l'une quelconque des revendications 1 à 11, ou la forme galénique de la revendication 12 ou 13, et un excipient pharmaceutiquement acceptable approprié.

15. Procédé de préparation de la composition d'insuline nanoparticulaire selon l'une quelconque des revendications 1 à 11, comprenant l'étape consistant à mettre les particules d'insuline en contact avec au moins un stabilisant de surface pendant un temps et dans des conditions qui suffisent pour fournir une composition d'insuline nanoparticulaire ayant une taille de particule moyenne effective de moins d'environ 5 microns.

16. Procédé selon la revendication 15, dans lequel ladite mise en contact comprend le broyage.

17. Procédé selon la revendication 15, dans lequel ladite mise en contact comprend l'homogénéisation.

18. Procédé selon la revendication 16, dans lequel ledit broyage est un broyage à haute énergie ou un broyage par voie humide.

19. Utilisation de la composition d'insuline nanoparticulaire selon l'une quelconque des revendications 1 à 11 pour la préparation d'une composition pharmaceutique pour l'insulinothérapie.
